# EUROPÄISCHE PATENTSCHRIFT

(11) **EP 0 975 350 B1**
(45) Veröffentlichungstag und Bekanntmachung des Hinweises auf die Patenterteilung: **01.08.2001**
(21) Anmeldenummer: 98919149.9
(22) Anmeldetag: 25.03.1998
(51) Int. Cl.: A61K 35/78

(54) **VERWENDUNG EINES EXTRAKTES VON ALCHEMILLA VULGARIS**
USE OF AN EXTRACT OF ALCHEMILLA VULGARIS
UTILISATION D'UN EXTRAIT D'ALCHEMILLA VULGARIS

(30) Priorität: 26.03.1997 DE 19712659
(43) Veröffentlichungstag der Anmeldung: 02.02.2000
(73) Patentinhaber: Weber & Weber GmbH & CO. KG, 82266 Inning (DE)
(72) Erfinder: FRANZ, Gerhard, D-93049 Regensburg (DE); PAPER, Dietrich, H., D-93142 Maxhütte-Haidhof (DE)
(74) Vertreter: Maiwald, Walter, Dr. Dipl.-Chem.
(86) Internationale Anmeldenummer: EP9801753
(87) Internationale Veröffentlichungsnummer: WO9842357

(56) Entgegenhaltungen:
- FR-A- 2 332 026

## Beschreibung

Die Erfindung betrifft die Verwendung eines Extraktes von Alchemilla vulgaris oder einer Fraktion desselben bzw. eines Lyophilisates davon oder eines oder mehrerer aktiven Inhaltsstoffe des Extraktes zur Herstellung eines Arzweimittels für die Behandlung von Krankheitszuständen, die durch die Hemmung der Angiogenese behandelbar sind.

Extrakte von Alchemilla vulgaris (Frauenmantel) sowie verschiedene phärmakologische Wirkungen dieser Extrakte sind bekannt.

Die Urtinktur von Alchemilla vulgaris stellt einen mit 43% (G/G) Ethanol gewonnenen Auszug aus Herba Alchemillae dar.

Die Urtinktur enthält Gerbstoffe vom Ellagitannin-Typ. Bekannt sind die Strukturen der dimeren Ellagitannine Agrimoniin., Laevigatin F und Pendunculagin. Ferner enthält die Urtinktur Flavonoide, Leukocyanidine und Kaffeesäurederivate (siehe Hänsel, R., Keller, K., Rimpler, H., Schneider, G. (Hrsg.), Hagers Handbuch Band 4, 5. Aufl., S. 161-166, Springer-Verlag Berlin, Heidelberg 1992; Ploss, E., Erkenntnismaterial der Kooperation Phytopharmaka, Alchemilla vulgaris L. Frauenmantel 1985).

Für die Flavonoide von Herba Alchemillae sind entzündungshemmende Wirkungen als Synthesehemmung von Prostaglandinen des Cyclooxygenase-Weges und spasmolytische Wirkungen bekannt. Kämpferol hemmt die Histaminfreisetzung aus Mastzellen. Für die Flavonoide und Leukocyanidine sind ferner kapillarabdichtende, antiödematöse Wirkungen beschrieben. An diesen Wirkungen können auch die haemostyptisch wirkenden Gerbstoffe beteiligt sein. Die haemostyptische Wirkung der Gerbstoffe wurde im Tierversuch bei peroraler Verabreichung von Herba Alchemillae nachgewiesen. Die Gerbstoffe wirken ferner adstringierend und dadurch reizlindernd, entzündungs- und sekretionshemmend an den Schleimhäuten des Magen-Darm-Traktes (Ploss, E., aaO.).

Bestimmte Flavonoidfraktionen aus Herba Alchemillae hemmen in vitro verschiedene Proteasen des Pankreas, Elastase, Trypsin und α-Chymotrypsin. Diese Enzyme können pathophysiologisch Bindegewebe schädigen oder auch Entzündungsmediatoren wie die Kinine aktivieren. Eine Gewebeschutzwirkung aufgrund der Proteasehemmungen wird diskutiert (Jonadet, M., et al, Flavonoides Extraits de Ribes Nigrum L. et d'Alchemilla vulgaris L., J. Pharmacol. 17, 1, 21-27 (1986)).

Toxische Wirkungen sind von Herba Alchemillae nicht bekannt (Ploss, E., aaO.).

Aufgrund der entzündungshemmenden Eigenschaften wird Herba Alchemillae bei Durchfallerkrankungen angewendet (Ploss, E., aaO.; Alchemillae herba, Monographie der Kommission E, BAnz. 173 vom 18.09.96). Ferner wird die Droge aufgrund ihrer spasmolytischen und haemostyptischen Wirkungen bei Menorrhagie und Metrorraghie therapeutisch innerlich eingesetzt (Ploss, E., aaO.). Hinzu kommen Anwendungen als Gurgellösung bei Entzündungen im Mund- und Rachenraum sowie die äußere Anwendung bei Geschwüren (Hänsel, R., et al., aaO.).

In FR-A-2332026 (Cariel, Leon) wird ein phytotherapeutisches Produkt auf der Basis eines Alchemilla-Extaktes, das zur topischen Behandlung von Bindegewebsläsionen geeignet ist und insbesondere die Resorption von Hautnarben, Striemen, Furchen, Falten und dergleichen durch Wirkung auf fibrilläre Strukturen (Fibroblasten etc.) fördert, beschrieben. Es bewirkt eine Wiederherstellung von arteriellem und vaskulärem Gewebe, z.B. Arteriosklerose. Insbesondere wurde eine Regeneration von Gefäßen im Bereich von weißen (nichtdurchbluteten) Hautnarben/-furchen gezeigt.

Überraschenderweise ist nunmehr festgestellt worden, daß Extrakte von Alchemilla vulgaris, insbesondere Lyophilisate davon, unerwartete neue pharmakologische Wirkungen zeigen, die weder vorbeschrieben noch durch die bekannten Inhaltsstoffe und bisherigen Indikationen der Droge nahegelegt sind.

Erfindungsgemäß betrifft die Erfindung die Verwendung eines Extraktes von Alchemilla vulgaris, vorzugsweise der Urtinktur, oder einer Fraktion desselben bzw. eines Lyophilisates davon, oder eines oder mehrerer aktiven Inhaltsstoffe des Extraktes zur Herstellung eines Arzweimittels für die Hemmung der Angiogenese, insbesondere bei Tumorerkrankungen, rheumatoider Arthritis und anderen chronisch entzündlichen Erkrankungen, Psoriasis, Retinopathien und Wurzelhautentzündungen der Zähne.

Besonders bevorzugt liegt die Dosierung der Urtinktur-Trockensubstanz zwischen 20 mg und 2 g pro Tag.

Erfindungsgemäß werden als Darreichungsformen bevorzugt Dragees, Hartgelatine-Kapseln, flüssige Zubereitungen der Urtinktur-Trockensubstanz als Oralia, topische Arzneimittel oder Injektabilia verwendet.

Hauptsächlich ist an den Einsatz bei Menschen gedacht, der Einsatz im Veterinär-Bereich ist aber ebenso möglich.

Die neuen pharmakologischen Wirkungen sind belegt worden durch die nachgewiesene Hemmung der Angiogenese in der Chorion-Allantois-Membran (CAM) des bebrüteten Hühnereies durch Lyophilisat der Urtinktur von Alchemilla vulgaris, wie oben näher spezifiziert.

Als Testpellet wurden dabei 10 *µ*l einer Lösung von 50 mg Lyophilisat in 1 ml Agaroselösung auf die CAM appliziert. Im Bereich des Testpellets erschien die Kapillardichte gegenüber dem Kontrollversuch geringer bis hin zu einer vollständigen Unterdrückung der Angiogenese als kapillarfreier Hof. Dieser Versuchsaufbau stellt ein anerkanntes Modell für die in-vitro-Überprüfung der Hemmung der Angiogenese dar und belegt die völlig unerwarteten, neuartigen, pharmakologischen Wirkungen der genannten Lyophilisate.

Der CAM-Test gehört zu einer Reihe von Modellversuchen, die Substanzen auf ihre Angiogenese-hemmende Wirkung für mögliche therapeutische Anwendungen zu prüfen. Der Vorteil des CAM-Testes liegt darin, daß er zu den in vivo-Versuchen gehört, die eine sicherere Aussage über klinische Relevanz zulassen als in vitro-Verfahren. In diesem in vivo-Test liegt das komplexe System der Angiogenese mit all seinen Zellfunktionen und Mediatoren vor, so daß eine vergleichsweise sichere Aussage über die Hemmwirkung der Angiogenese möglich ist. Der Test ist als Screening-Verfahren zur Ermittlung von Substanzen mit Angiogenese-hemmenden Eigenschaften anerkannt (Svahn, C.M., M. Weber, C. Mattsson, K. Neiger, M. Palm Carbohydr. Polym. 18. 9-16 (1992) ; Hahnenberger R., A.M. Jakobsen, A. Ansari, T. Wehler, C.M. Svahn, U. Lindahl, Glycobiology 3, 567-573 (1993); und Gagliardi, A., H. Hadd, D.C. Collins, Cancer Res. 52, 5073-5075 (1992)).

Die Angiogenese ist ein physiologischer differenzierender Gewebeprozeß in der Embryonalentwicklung, nach der weiblichen Regelblutung und bei der Wundheilung. Diese Differenzierung geht von Kapillaren aus, in dem die Basalmembran stellenweise aufgelöst wird, Endothelzellen migrieren und proliferieren, sich zu einer Röhre zusammenschließen und mit benachbarten Proliferationsstellen eine Schleife bilden. Die Basalmembran wird an den neu entstandenen Gefäßen ausgebildet. Dieser Prozeß ist einer Steuerung durch einander antagonisierende Mediatoren unterworfen. Zu den Angiogenese-stimulierenden Faktoren zählen der Acidic Fibroblast Growth Factor (FGF-1), der Basic Fibroblast Growth Factor (FGF-2), der Vascular Endothelial Growth Factor (VEGF), Interleukin 1 α (IL 1α) u.a.. Endogene Inhibitoren stehen diesen stimulierenden Faktoren gegenüber und verhindern beim Gesunden die Angiogenese.

Bei verschiedenen Erkrankungen spielt in der Pathogenese die Gefäßneubildung eine Rolle. Dazu gehören vor allem Tumorerkrankungen. Sowohl das Wachstum eines soliden Tumors als auch die Metastasierung ist abhängig von der Angiogenese im Tumorgewebe. Weitere Beispiele für Erkrankungen, bei denen die Angiogenese eine pathogenetische Rolle spielt, sind oben bereits genannt.

Wirksame und nebenwirkungsarme Angiogenese-Inhibitoren stellen heute noch eine therapeutische Lücke dar, da noch kein zur Anwendung am Menschen zugelassener Angiogenese-Hemmstoff zur Verfügung steht. Es wurden zwar schon verschiedene Angiogenese-Inhibitoren, z.B. Sumarin, klinisch geprüft, aber der therapeutische Nutzen wird aufgrund toxischer Wirkungen in Frage gestellt.

Mit der pharmakologischen Prüfung des Lyophilisates der Urtinktur von Alchemilla vulgaris an der CAM des Hühnerembryos trat völlig überraschend die starke Angiogenese-Hemmwirkung eines pflanzlichen Arzneistoffes auf, von dem bisher keine Nebenwirkungen bekannt geworden sind.

Neue Therapiestrategien bei der rheumatoiden Arthritis werden in Angiogenesehemmstoffen gesucht, da beim Entzündungsprozeß die synoviale Proliferation mit einer Neovaskularisierung verbunden ist. Die Unterdrückung der Proliferation von Endothelzellen kann als wichtiges Therapieziel angesehen werden, da hiermit auch eine Reduzierung des pathologisch-immunologischen Prozesses zu erwarten ist.

Verträgliche mittlere Tagesdosen von Herba Alchemillae reichen in der Phytotherapie bis zu 10 g, worin allerdings noch keine Höchstdosis zu sehen ist. Mögliche Dosierungen der Urtinktur-Trockensubstanz liegen daher bevorzugt zwischen 20 mg und 2 g.

Als Darreichungsformen kommen bevorzugt Dragees, Hartgelatine-Kapseln, flüssige Zubereitungen der Urtinktur-Trokkensubtanz als Oralia, topische Arzneimittel oder Injektabilia in Frage.

## Patentansprüche

1. Verwendung eines Extraktes von Alchemilla vulgaris oder einer Fraktion desselben bzw. eines Lyophilisates davon, oder eines oder mehrerer aktiven Inhaltsstoffes des Extraktes zur Herstellung eines Arzneimittels für die Behandlung von Krankheitszuständen, die durch die Hemmung der Angiogenese behandelbar sind.

2. Verwendung nach Anspruch 1 zur Herstellung eines Arzneimittels für die Hemmung der Angiogenese bei Tumorerkrankungen, rheumatoider Arthritis und anderen chronisch entzündlichen Erkrankungen, Psoriasis, Retinopathien und Wurzelhautentzündungen der Zähne.

3. Verwendung nach Anspruch 1 oder 2,
**dadurch gekennzeichnet,** daß als Extrakt die Urtinktur verwendet wird.

4. Verwendung nach Anspruch 3,
**dadurch gekennzeichnet,** daß die Dosierung der Urtinktur-Trockensubstanz zwischen 20 mg und 2 g pro Tag liegt

5. Verwendung nach einem der vorangehenden Ansprüche,
**dadurch gekennzeichnet,** daß als Darreichungsformen Dragees, Hartgelatine-Kapseln, flüssige Zubereitungen der Urtinktur-Trockensubstanz als Oralia, topische Arzneimittel oder Injektabilia verwendet werden.

## Claims

1. The use of an extract of Alchemilla vulgaris or a fraction thereof or a lyophilisate thereof or one or several active ingredients of the extract, for the preparation of a drug for the treatment of diseases which can be treated by the inhibition of angiogenesis.

2. Use according to claim 1 for the preparation of a drug for the inhibition of angiogenesis in tumor diseases, rheumatoid arthritis, and other chronic inflammatory diseases, psoriasis, retinopathies, and periodontal inflammations.

3. Use according to claims 1 or 2,
**characterized in** that the primary tincture is used as extract.

4. Use according to claim 3,
**characterized in** that the dosage of the primary tincture dry substance is between 20 mg and 2 g per day.

5. Use according to any one of the preceding claims,
**characterized in** that the preparation can be administered in the form of dragees, hard gelatine capsules, liquid preparations of the primary tincture dry substance as oral medicines, topical drugs or injectable drugs.

## Revendications

1. Utilisation d'un extrait d'Alchemilla vulgaris ou d'une fraction de celui-ci ou respectivement d'un lyophilisat de celui-ci ou d'un ou plusieurs ingrédients actifs de l'extrait pour la fabrication d'un médicament destiné au traitement des maladies pouvant être soignées par inhibition de l'angiogenèse.

2. Utilisation selon la revendication 1 pour fabriquer un médicament inhibiteur de l'angiogenèse dans les affections tumorales, l'arthrite rhumatoïde et autres affections inflammatoires chroniques, le psoriasis, les rétinopathies et les inflammations des gencives.

3. Utilisation selon la revendication 1 ou 2, caractérisée en ce que la teinture mère est utilisée à titre d'extrait.

4. Utilisation selon la revendication 3, caractérisée en ce que la dose à administrer de matière sèche de teinture mère se situe entre 20 mg et 2 g par jour.

5. Utilisation selon l'une des revendications précédentes, caractérisée en ce que les formes de présentation utilisées sont les comprimés, les capsules de gélatine solide, des préparations liquides de matière sèche de teinture mère pour la prise orale, les médicaments topiques ou les injectables.
